# EUROPEAN PATENT SPECIFICATION

(11) **EP 2 692 355 B1**
(45) Date of publication and mention of the grant of the patent: **16.06.2021**
(21) Application number: 13003778.1
(22) Date of filing: 30.07.2013
(51) Int. Cl.: A61K 38/40, A61P 3/00

(54) **Apolactoferrin for the treatment of iron accumulation diseases**
Apolactoferrin zur Behandlung von Eisenspeicherkrankheiten
Apolactoferrin pour le tratiement des maladies liées à l'accumulation de fer

(30) Priority: 01.08.2012 IT MI20121356
(43) Date of publication of application: 05.02.2014
(73) Proprietor: TDC Technology Dedicated to Care S.r.l., 20125 Milano (IT)
(72) Inventor: Biancardi, Biagio, 80127 Napoli (IT)
(74) Representative: Trupiano, Federica

(56) References cited:
- EP-A1- 1 917 972
- WO-A2-2010/007494
- LEGRAND D ET AL: "Lactoferrin; Lactoferrin: a modulator of immune and inflammatory responses", CMLS CELLULAR AND MOLECULAR LIFE SCIENCES, BIRKHÄUSER-VERLAG, BA, vol. 62, no. 22, 1 November 2005 (2005-11-01), pages 2549-2559, XP019200888, ISSN: 1420-9071, DOI: 10.1007/S00018-005-5370-2
- None

## Description

### STATE OF THE ART

The object of the present invention is a pharmaceutical, dermatological or cosmetic preparation based on a mixture of lactoferrin and apolactoferrin suitable to be used in the treatment of diseases characterized by iron accumulation.

Iron is an essential element for the life of most living organisms, included humans and most of the bacteria, thanks to its ability of acting both as a donor and acceptor of electrons. However, this characteristic thereof is exhibited in a positive way when iron is present in the form of protein ligand. In case wherein iron is present as a free element within the cell, it may possibly result toxic for the living organisms.

Iron is a "heavy metal", and the living organisms are highly sensitive to metals in general, and in particular to Fe, whose dosage is finely tuned from a metabolic point of view. Then iron shortage or accumulation result to be dangerous in the same way to the living organisms.

The origin of its potential toxicity, lies in the fact that excess iron tends to accumulate into tissues, resulting bio-accumulative. An excessive amount of this metal accumulated into the organism may result in a high toxicity, since the free iron ions react with peroxides and produce free radicals which, being highly reactive, may cause damages to DNA, proteins, lipids and other cell components. Therefore, an iron accumulation into the cells may cause significant damages to the organism. Lactoferrin, well known since long time and recently re-evaluated for immunomodulating and anti-infections properties thereof as well as largely described in literature, is a glycoprotein with antimicrobial and iron-transport activities provided with multiple bioactive properties connected with its capacity of chelating iron. It is a natural, endogenous chelant, and is involved in several biological mechanisms.

Lactoferrin is a globular protein with a peculiar tri-dimensional structure. It is organized in two globular lobes, the N-terminal lobe and the C-terminal lobe, connected by an alpha-helix structure. Both protein lobes are constituted by two domains. The protein is structured in such a way to have available a single binding site to iron for each lobe.

Each lactoferrin molecule can bind with a maximum of two ferric ions and based on such a saturation it may exist in three distinct forms: apolactoferrin (without iron), monoferric lactoferrin (bound to a single ferric ion) and hololactoferrin (that binds two ferric ions). The known lactoferrin presents a Fe saturation ratio equal to 25 mg of Fe to 100 g of lactoferrin.

The protein activity is conserved even in acid environments and in presence of proteolytic enzymes, including those secreted by microorganisms.

In the Patent Application filed as N. MI2008A001285 published as WO2010007494, a composition based on lactoferrin vehiculated in nanolipids and the use thereof for the treatment of skin diseases which are characterized by accumulation of heavy metals, is described. Also, uses of the lactoferrin form without iron are known, that is of apolactoferrir EP1917972 discloses the use of apolactoferrin for the treatment of diseases caused by a heavy metal such as iron

For example, JP2011093893 describes the use of apolactoferrin in the treatment of allergies. JP2010229118 describes a product based on proteins derived from milk such as lactoferrin, apolactoferrin and enzymatic casein, capable of inhibiting the lipase and thus advantageously employed as a weight loss adjuvant. WO2010005012 describes an apolactoferrin - based composition together with at least one additional ingredient selected among a product-binding glycated agent, an antioxidant substance and an antimicrobial. The described composition can be used in the formulation of several products, e.g. food products, cosmetics, detergents, perfumes and still others.

Thus the use of apolactoferrin as antimicrobial (WO2010005012), as weight loss adjuvant (JP2010229118), and antihistamine (JP2011093893) is known. Furthermore, WO2008032847 describes a topical preparation for the skin with antibacterial properties containing apolactoferrin at a weight concentration of 1% and with an iron binding level of about 5%.

JP2008063303 describes a mouthwash with antibacterial properties containing apolactoferrin at a weight concentration of 1% and with an iron binding level of about 5%.

It is therefore clear that apolactoferrin can play a key role in medical practice, but problems exist regarding the chemical physical nature thereof that limit its use, in spite of its high free iron binding capacity (1 mg Fe/100 g apolactoferrin).

The two apolactoferrin lobes, i.e. the native protein not bound to iron, can take an open or close conformation, and pass from one form to the other as a result of the energetic levels characterizing the two states. The N-terminal lobe in apolactoferrin takes an open conformation, whereas the C-terminal lobe remains closed. It is assumed that the iron binding occurs in the open form of the apo-protein, because it allows an easier access to free or complexed Fe3+ ions. Moreover, the Fe binding occurs at neutral or basic pH and its release at acidic pH. Apolactoferrin thus has a high capacity of iron capture into the lobes, but the state change from the open to the close conformation occurs in an extremely rapid way thus making the protein easily degradable and denaturable. This aspect relative to the easy apolactoferrin degradation and denaturation has prevented, until now, to take the full advantage of potential applications in cosmetic and pharmaceutical fields.

### OBJECTS OF THE INVENTION

Object of the present invention is to provide a composition to be used in the treatment of diseases characterized by metallic iron accumulation.

Also object of the present invention is to provide a composition for the treatment of skin diseases iron accumulation such as ecchymotic wounds.

It is also object of the present invention to provide a pharmaceutical composition for topical use for the treatment of cutaneous dyschromias due to hemosiderin or ferritin accumulation.

Even object of the present invention is to provide a cosmetic composition for the treatment of cellulitis.

Still object of the invention is to provide a pharmaceutical composition for the treatment of neurodegenerative diseases characterized by injuries caused by an iron-dependent inflammatory process.

### DESCRIPTION OF THE INVENTION

These and further other objects and the relative advantages that will be better explained in the following description, are achieved using a composition comprising apolactoferrin, mixed with lactoferrin, in addition to common excipients and additives to be used for the treatment of diseases characterized by iron accumulation, in particular skin diseases.

This hereinafter called apolactoferrin-based composition is used, according to the invention, vehiculated into nanolipids.

It was surprisingly found that the apolactoferrin-based composition presents peculiar characteristics and allows to obtain remarkable results in the treatment of iron accumulation diseases, in addition showing an unexpected effect with respect to the action of the lactoferrin alone.

Furthermore, as the above mentioned composition comprises, in addition to apolactoferrin, also a certain amount of lactoferrin, it shows a synergic effect if used according to the invention, with respect to the effect shown by the components thereof (lactoferrin and apolactoferrin) if taken individually.

Thus the present invention goes beyond the limits of the known art, especially with respect to the protein degradation and denaturation readiness in its form without iron, providing a composition wherein apolactoferrin is in the suitable conformation for accepting Fe ions but, at the same time, it does not result to be neither denatured nor degraded. Thus the composition takes advantage of the chelating properties of apolactoferrin for the treatment, according to the present invention, of diseases characterized by iron accumulation.

With the term "iron accumulation diseases", it is meant those diseases wherein Fe results to be involved both as a triggering factor and a factor correlated to basic disease. In particular, apolactoferrin is advantageously used, according to the invention, for both topical and systemic treatment of diseases such as cutaneous dyschromias, neurodegenerative diseases, cellulitis, iron-dependent vein ulcers.

The composition is advantageously used according to the present invention for the treatment of skin diseases characterized by iron accumulation, in particular in the treatment of skin diseases such as ecchymotic wounds, and more in particular for the treatment of cutaneous dyschromias correlated thereto.

Still according to the invention, the composition is advantageously used for the treatment of ecchymotic pigmentations of different origin.

The composition can be used in the pharmaceutical, cosmetic or cosmeceutic field, and is in particular intended for topical use.

With the term "topical" it is meant all those treatments which provide for a local treatment, i.e. limited to a specific body region, such as for example the sublingual, mesotherapeutic administration, and the others well known in the state of the art. Thus the object of present invention is the use as defined in the claims for the treatment of iron accumulation diseases in the cosmetic, cosmeceutic, dermatologic or pharmaceutical field.

The same composition can be advantageously used, according to the invention, in association with nanolipids, showing the same advantages with respect to the compositions according to the known art.

Object of the present invention is also an apolactoferrin-based pharmaceutical composition, mixed with lactoferrin for the treatment, according to the invention, of skin diseases characterized by iron accumulation and it can possibly be added with pharmaceutically acceptable salts and/or additives.

With the term "pharmaceutical composition or formulation" it is meant those compositions or formulations comprising apolactoferrin, mixed with lactoferrin, as the active ingredient, in addition to other components and/or additives compatible with the pharmaceutical practice.

With the term "addition of pharmaceutically acceptable salts" it is meant to refer herein to all those salts that are compatible with the pharmaceutical practice from a biological, preparation and formulation point of view.

In particular, the above composition is advantageously used, according to the present invention, for the preparation of a medicament for the treatment of cutaneous dyschromias due to ferritin and/or hemosiderin accumulation.

The above mentioned formulations or compositions are suitable for the topical administration of the active ingredients contained thereto.

The topical formulations for the treatment of the diseases characterized by iron accumulation are, e.g., creams, lotions, mousses, sprays, emulsions, gels, unguents, impregnated bandages and the like, compatible with the preparation according to the methods commonly known in the state of the art.

The formulation used according to the invention comprises a variable weight amount of apolactoferrin and comprised between 0.1% and 20% relative to the weight of the composition, in particular it is comprised between 0.3 and 15%, still relative to the total weight of the composition.

The formulation according to the invention comprises, as active substance, a mixture of lactoferrin and apolactoferrin, present in the following concentrations:
- Lactoferrin in a concentration range comprised between 1 and 20 wt%, preferably between 3 and 12 wt%, still more preferably between 5 and 9 wt%, relative to the total weight of the composition.
- Apolactoferrin in a concentration range comprised between 0.1 and 20 wt%, preferably between 0.3 and 15 wt% relative to the total weight of the composition, the above mentioned concentration being referred to the weight percent relative to the total weight of the composition, and being intended for the use in the treatment of iron accumulation diseases.

According to a preferred embodiment of the invention, lactoferrin is present at a concentration of 0.6 wt% and apolactoferrin at a concentration of 3 wt% relative to the total weight of the composition.

According to a particularly preferred embodiment of the invention, the formulations for the administration in mesotherapeutic form comprise apolactoferrin, mixed with lactoferrin, as the active ingredient, in a concentration range comprised between 0.5 and 20 wt%, preferably between 2 and 13 wt%, still more preferably between 5 and 9 wt% relative to the total weight of the composition.

Excipients used in the formulations according to the invention may be those known to the person skilled in the art, preferably in the preparation of creams, unguents, impregnated bandages can be used those selected between PEG 400, PEG 1500, PEG 4000, water, sorbitol, vegetable glycerol, allantoin, cetyl alcohol, stearyl alcohol, phenoxyethanol, tocopherol acetate.

According to the present invention, the mixture of lactoferrin and apolactoferrin, as the active ingredient, is associated to nanolipids.

With the term "nanolipids" it is meant to refer to those particles whose the structure is analogous to those of cell membranes, in particular to those preferably with a diameter below 250 nm. They are mainly composed of natural phospholipids organized in a double-layer.

The mixture based on lactoferrin and apolactoferrin for its use, according to the invention, in the treatment of iron accumulation diseases, goes beyond the limits of the known art since the active substance, i.e. apolactoferrin mixed with lactoferrin, shows unexpected effects in the Fe saturation. The unexpected and improved saturation permits the formulations, when used according to the invention, to increase the Fe adsorption when it is in the free state inside the cell (and thus when it is in conditions of possible cell damage) and to aid the healing process of all those diseases where Fe accumulation is found and dangerous.

As already said, the composition based on a mixture based on lactoferrin and apolactoferrin, for its use in the treatment of diseases characterized by iron accumulation, is advantageously associated to vehicles such as nanolipids, to aid the penetration and diffusion of active ingredients contained therein. A further advantage of the composition vehiculated as described above, is represented in that the vehiculation into nanolipids, can result to be useful in its stabilization and protection due to a possible denaturation.

In particular in case of iron accumulation diseases, the composition based on a mixture of lactoferrin and apolactoferrin vehiculated in this way, can reach, in a significant amount relative to the initially administered amount and in an intact form, the accumulations of such a metal and it is thus capable of performing its chelating action by subtracting iron from cells and thus removing the damage source.

It is clear that the pharmaceutical composition with chelating activity comprises apolactoferrin mixed with lactoferrin, for the treatment of skin impurities and blemishes connected with iron accumulation according to the invention, and is characterized by excellent pharmacokinetic and bioavailability parameters.

The composition can be used, for example, for the treatment of skin impurities and dyschromias blemishes from harmful drosses, ecchymotic pigmentations, and toxic accumulations. Said composition guarantees an effective depurative and bleaching effect thanks to the chelating activity of the components thereof, in particular thanks to the activity carried out by peculiar active ingredients (apolactoferrin mixed with lactoferrin). Said chelating activity guarantees a rapid removal of toxic accumulations of exogenous and endogenous origin.

The composition, for the surprising characteristics thereof, removes the iron dermal accumulations even in particular conditions, e.g. conditions where the iron accumulation results from the stimulation and production of free radicals that aid and accelerate the aging effect related to the dermo-epidermal structures.

The use of the composition according to the invention is also particularly favorable in obtaining a reduction of the brownish color due to the ferritin iper-accumulation in chronic venous insufficiency and following the sclerosant therapy.

The composition could also be used for iron-dependent vein ulcers and also in cellulitis treatment.

The formulation for the topical use can be used, according to the invention, even several times a day, preferably 2 times a day, distributed within 24 hours.

All the concentrations indicated in the present application have to be considered as weight percent of each active ingredient relative to the total weight of the formulation/ composition.

The composition thus contains apolactoferrin as the active ingredient mixed with lactoferrin, and it results to be selective in excess Fe chelation and is completely atoxic. Therefore, by using the composition according to the invention, it is achieved a rapid removal of iron accumulations, a drastic and significant reduction of free radical formation with the resulting reduction of the oxidative stress responsible for several diseases, e.g. skin aging and cellulitis. In addition, the selective chelating activity provided by the composition on the hemosiderin and ferritin deposits, makes the composition particularly effective and active in the reabsorption of ecchymotic pigmentations due to accidental traumas or surgery activities. The high selectivity of the composition makes the use thereof favorable for the treatment of cutaneous dyschromias due to sclerosant therapy and for those deriving from chronic venous insufficiency.

The present invention is better illustrated by means of the examples, according and not according to the invention, reported in the following, in no way being limitative.

### EXAMPLE 1 - Formulations

The formulation comprises: Apolactoferrin in the range from 0.5% to 20% in saline in liquid formulation for mesotherapeutic uses. Apolactoferrin in the range from 0.5% to 20%, Lactoferrin from 0.5% to 20% in saline in liquid formulation for mesotherapeutic uses.

Apolactoferrin from 0.5% to 20%, Lactoferrin from 0.5% to 20%, Allantoin 1%, Tocopherol acetate 0.5%, Water, Sorbitol, Vegetal glycerol, PEG 400, PEG 1500, PEG 4000. In the formulations of creams, impregnated bandages or cream impregnated gauzes. Apolactoferrin from 0.5% to 5% in nanolipids, Allantoin, Tocopherol acetate, Water, Sorbitol, Vegetal glycerol, PEG 400, PEG 1500, PEG 4000. Apolactoferrin in nanolipids from 0.5% to 5%, Lactoferrin in nanolipids from 0.5 to 5%, Allantoin, Tocopherol acetate, Water, Sorbitol, Vegetal glycerol, PEG 400, PEG 1500, PEG 4000, in the formulation of creams/ ointments, impregnated bandages or cream impregnated gauzes.

### EXAMPLE 2 - In-vitro test

Three different compositions based on Apolactoferrin (A), on a mixture of Apolactoferrin and Lactoferrin (AL) and on Lactoferrin (L) have been tested in-vitro on histologic samples from porcine ears. Object of the study is to evaluate the differences in iron chelating ability between the three different compositions under the same experimental conditions.

The histologic sample has been treated at a fixed concentration of 1% of A (Apolactoferrin alone), AL (mixture of Apolactoferrin and Lactoferrin) and L (Lactoferrin alone), and the iron chelating ability has been tested.

A, AL, and L compositions contained 20 g of apolactoferrin, 10 g of apolactoferrin and 10 g of lactoferrin, 20 g of lactoferrin, respectively. Said compositions are applied on the sample and incubated at 37°C for 20 h. The applied compositions have been recovered at different times (1h, 4h, 8h, 16h and 20h) and the amount of the active ingredient has been determined by HPLC.

The results have surprisingly demonstrated that A and AL compositions have a Fe chelating ability greater than lactoferrin as is. As a matter of fact, the amounts of active ingredient recovered from the sample treated with A and AL are drastically lower than the sample treated with lactoferrin alone and therefore A and AL have a surprising bioavailability.

Above mentioned data comes from an in-vitro test carried out at the Pharmacy Faculty of Naples, where the application of the Apolactoferrin and Lactoferrin mixture, into suitable excipients, on porcine ears demonstrated a higher chelating activity towards iron with a higher bioavailability to reach the target site.

## Claims

1. Composition comprising mixture of apolactoferrin and lactoferrin vehiculated into nanolipids, which are particles, composed of natural phospholipids organized in a double-layer, having a structure analogous to the cell membranes, with a diameter of less than 250 nm, for use in the treatment of iron accumulation diseases.

2. Composition for use according to claim 1, **characterized in that** said iron accumulation diseases are selected from cutaneous dyschromias, ecchymotic pigmentations, cellulitis, iron-dependent vein ulcers, neurodegenerative diseases.

3. Use of a composition comprising apolactoferrin vehiculated into nanolipids, as said nanolipids are defined in claim 1, in the treatment of skin aging.

4. Composition for use according to claim 1 for the use in topical treatment of iron accumulation diseases selected from cutaneous dyschromias, ecchymotic pigmentations, iron-dependent vein ulcers.

5. Use of cosmetic comprising apolactoferrin vehiculated into nanolipids, as said nanolipids are defined in claim 1, in topical or mesotherapeutic treatment of cellulitis.

## Patentansprüche

1. Zusammensetzung, umfassend eine Mischung von Apolactoferrin und Lactoferrin, die in Nanolipide eingebracht ist, welche Partikel sind, die aus natürlichen Phospholipiden bestehen, die in einer Doppelschicht organisiert sind, die eine Struktur analog zu der Zellmembran haben, mit einem Durchmesser von weniger als 250 nm, zur Verwendung bei der Behandlung von Eisenanreicherungserkrankungen.

2. Zusammensetzung zur Verwendung gemäß Anspruch 1, **dadurch gekennzeichnet, dass** die Eisenanreicherungserkrankungen ausgewählt sind aus kutanen Dyschromien, ekchymotischen Pigmentierungen, Cellulitis, eisenabhängigen Venengeschwüren, neurodegenerativen Erkrankungen.

3. Verwendung einer Zusammensetzung enthaltend Apolactoferrin, die in Nanolipide eingebracht ist, wobei die Nanolipide wie in Anspruch 1 definiert sind, bei der Behandlung von Hautalterung.

4. Zusammensetzung zur Verwendung gemäß Anspruch 1, zur Verwendung bei der topischen Behandlung von Eisenanreicherungserkrankungen ausgewählt aus kutanen Dyschromien, ekchymotischen Pigmentierungen, eisenabhängigen Venengeschwüren.

5. Verwendung eines kosmetischen Mittels enthaltend Apolactoferrin, das in Nanolipide eingebracht ist, wobei die Nanolipide wie in Anspruch 1 definiert sind, zur der topischen oder mesotherapeutischen Behandlung von Cellulitis.

## Revendications

1. Composition comprenant un mélange d'apolactoferrine et de lactoferrine véhiculé dans des nano lipides, qui sont des particules, composées de phospholipides naturels organisés en une double couche, ayant une structure analogue aux membranes cellulaires, avec un diamètre inférieur à 250 nm, pour une utilisation dans le traitement des maladies liées à l'accumulation de fer.

2. Composition à utiliser selon la revendication 1, **caractérisée en ce que** lesdites maladies liées à l'accumulation de fer sont choisies parmi les dyschromies cutanées, les pigmentations ecchymotiques, la cellulite, les ulcères veineux dépendants du fer, les maladies neurodégénératives.

3. Utilisation d'une composition comprenant de l'apolactoferrine véhiculée dans des nano lipides, tels que lesdits nano lipides sont définis dans la revendication 1, dans le traitement du vieillissement cutané.

4. Utilisation d'une composition selon la revendication 1 pour l'utilisation dans le traitement topique des maladies liées à l'accumulation de fer choisies parmi le dyschromies cutanées, les pigmentations ecchymotiques, les ulcères veineux dépendants du fer.

5. Utilisation d'un cosmétique comprenant de l'apolactoferrine véhiculée dans des nano lipides, tels que lesdits nano lipides sont définis dans la revendication 1, dans le traitement topique ou mésothérapeutique de la cellulite.
